# NOUVEAU FASCICULE DE BREVET EUROPEEN

(11) **EP 1 674 475 B2**
(45) Date de publication et mention de la décision concernant l'opposition: **05.11.2014**
(45) Mention de la délivrance du brevet: 02.03.2011
(21) Numéro de dépôt: 05292670.6
(22) Date de dépôt: 14.12.2005
(51) Int. Cl.: C07H 15/04, A23L 1/09

(54) **Procédé de fabrication d'une poudre contenant des particules cristallines de glucopyranosyl-alditols**
Verfahren zur Herstellung von Pulver enthaltenden Kristallpartikeln von Glukopyranosyl-alditolen
Process for the preparation of a powder containing glucopyranosyl-alditols cristalline particules

(30) Priorité: 21.12.2004 FR 0413660
(43) Date de publication de la demande: 28.06.2006
(73) Titulaire: ROQUETTE FRERES, 62136 Lestrem (FR)
(72) Inventeur: Duflot, Pierrick, 62136 La Couture (FR); Zhou, Liuming, Macomb, IL 61455 (US)
(74) Mandataire: Cabinet Plasseraud

(56) Documents cités:
- EP-A- 1 172 370
- EP-A- 1 284 102
- WO-A-00/64916

## Description

La présente invention est relative à un procédé de fabrication continue d'une poudre contenant des particules cristallines de glucopyranosyl-alditols.

On entend par « glucopyranosyl-alditols » au sens de l'invention, des composés choisis dans le groupe constitué du 1-O-α-D-glucopyranosyl-D-mannitol (1,1 GPM), du 6-O-α-D-glucopyranosyl-D-sorbitol (1,6 GPS) et du 1-O-α-D-glucopyranosyl-D-sorbitol (1,1 GPS).

Ces glucopyranosyl-alditols sont le résultat de l'hydrogénation de l'isomaltulose et du tréhalulose, eux-mêmes produits par l'isomérisation du saccharose par des activités enzymatiques isolées de microorganismes particuliers.

Les plus connus de ces microorganismes appartiennent aux genres *Protaminobacter* (telle *P*. *rubrum* CBS 547.77), *Erwinia* (telle *E. rhapontic*i NCPPB 1578), *Serratia* (telle *S. marcescens* NCIB 8285 ou *S*. *plymouthica* ATCC 15928), *Leuconostoc* (telle *L*. *mesenteroides* NRRL B-521f ou ATCC 10830a), *Pseudomonas* (telle *P. mesoacidophila* MX-45), *Agrobacterium* (telle A. *radiobacter* MX-232*)*, *Klebsiella* ou *Enterobacter sp.*

L'isomaltulose et le tréhalulose peuvent être hydrogénés en mélange ou séparément (la séparation de l'isomaltulose du tréhalulose étant réalisée grâce notamment à des techniques de cristallisation ou des techniques chromatographiques).

Bien que l'hydrogénation de l'isomaltulose seul doive générer en théorie un mélange équimolaire de 1,6 GPS et de 1,1 GPM, il a été montré qu'en réalité la proportion de chacun des deux isomères est comprise entre 43 et 57 %. Ces mélanges de 1,6 GPS et de 1,1 GPM sont généralement commercialisés sous leur forme cristallisée, sous les noms de marque ISOMALT® ou PALATINIT® .

De la même façon, l'hydrogénation du tréhalulose seul conduit à l'obtention d'un mélange de 1,1 GPM et de 1,1 GPS.

Les glucopyranosyl-alditols, seuls ou en mélange, voire en proportions relatives variables, sont généralement utilisés comme édulcorants à faible pouvoir calorique. L'ISOMALT® , par exemple, est employé en confiserie, dans les produits de boulangerie, dans les desserts, dans les confitures, le chocolat, le chewing-gum et dans de nombreux articles diététiques.

Les procédés classiques de cristallisation des mélanges des trois glucopyranosyl-alditols nécessitent généralement des équipements spécifiques combinant l'évaporation sous vide, la cristallisation proprement dite et le séchage (Carbohydrates in Industrial Synthesis, edited by M.A. CLARKE, Verlag Dr Albert Bartens KG, Berlin, 1992, page 48).

Cependant, ces procédés présentent comme inconvénients qu'il est difficile de rendre l'appareillage simple à utiliser et qu'il est également difficile de travailler en continu en raison de la longue durée nécessaire à l'obtention des mélanges de cristaux.

En effet, ces procédés de cristallisation ne permettent pas d'épurer en une seule étape la totalité des glucopyranosyl-alditols de la solution mère. Pour optimiser le rendement de cristallisation, il faut donc procéder à deux ou trois étapes de cristallisation successives.

Or, il est connu que le 1,1 GPS est difficilement cristallisable en lui-même, et: qu'il perturbe également la cristallisation du 1,6 GPS et du 1,1 GPM. L'enrichissement des eaux mères en 1,1 GPS au cours des étapes successives de cristallisation complique ainsi le procédé de cristallisation de ces deux isomères et en réduit l'efficacité.

Pour remédier à cet inconvénient, le brevet EP 1.173.453 décrit un procédé de cristallisation des glucopyranosyl-alditols, consistant à mettre en contact un liquide, contenant un ou plusieurs glucopyranosyl-alditols dissous, avec des particules fines solides contenant un ou plusieurs desdits glucopyranosyl-alditols en suspension dans un gaz.

Cependant, ce procédé rend obligatoire des étapes supplémentaires d'élimination substantielle de la composante solvant du mélange ainsi obtenu par introduction d'air chauffé à une température pouvant atteindre 250°C, et de traitement de la matière glucopyranosyl-alditol qui en résulte à une sédimentation de manière à récupérer une composition de matière essentiellement solide comprenant une multitude de cristaux dudit ou desdits glucopyranosyl-alditols.

Il faut encore procéder à un séchage supplémentaire de ladite composition de cristaux de glucopyranosyl-alditols, puis à un broyage et un tamisage pour enfin obtenir un produit présentant des particules de glucopyranosyl-alditols contenant une multitude de microcristaux agglomérés entre eux de manière aléatoire.

Une autre solution à l'obtention du mélange solide cristallin des glucopyranosyl-alditols est décrite dans le brevet EP 1.172.370.

Le procédé décrit dans ce brevet, qui permet d'obtenir un mélange cristallin solide comprenant 20 à 70 % de 1,1 GPM, 23 à 70 % de 1,6 GPS et de 2 à 25 % de 1,1 GPS, consiste à introduire en continu un mélange hydrogéné d'isomaltulose et de tréhalulose (préparé à partir de sucre de canne) avec des amorces cristallines de glucopyranosyl-alditols dans un extrudeur muni d'une zone de refroidissement fine et longue.

Plus précisément, le procédé comprend :
- l'addition de germes cristallins de glucopyranosyl-alditols à une température inférieure au point de fusion desdits germes cristallins de glucopyranosyl-alditols dans une solution aqueuse de glucopyranosyl-alditols ayant 2 à 10 % en poids d'humidité, et
- le refroidissement et le pétrissage de ladite solution aqueuse de glucopyranosyl-alditols en présence de germes cristallins, de manière à produire un magma de glucopyranosyl-alditols qui est extrudé continuellement depuis une buse.

Des étapes ultérieures de désintégration grossière, de maturation, de séchage, etc. sont également mentionnées dans le cadre de la production d'un produit final poudreux.

Cependant, dans ce procédé, le refroidissement est utilisé pour effectuer la cristallisation en augmentant le degré de sursaturation de la solution. Il est donc nécessaire de réduire la viscosité en chauffant la solution ayant une forte concentration en glucopyranosyl-alditols à une température élevée afin que les germes cristallins soient dispersés de façon homogène en une courte durée.

Ce procédé présente l'inconvénient d'absorber énormément d'énergie car il nécessite la formation d'une « masse cuite », c'est-à-dire une substance sursaturée contenant des germes cristallins et présentant une viscosité qui est abaissée par chauffage à température élevée (jusqu'à 140°C) et à pression élevée.

Il est également nécessaire d'utiliser cet appareil de chauffage particulier pour empêcher la détérioration d'une capacité de mélange et de dispersion résultant d'une augmentation de la viscosité due à un abaissement de la température et il est critique de conserver une faible viscosité afin de disperser et de mélanger les germes cristallins en une durée limitée.

D'autre part, le procédé nécessite également de contrôler finement le rapport 1,1 GPM / 1,6 GPS / 1,1 GPS des amorces de manière à ce qu'il soit identique à celui de la solution de glucopyranosyl-alditols introduite dans l'extrudeur.

EP 1.284.102 décrit un procédé permettant d'obtenir une composition solide d'un mélange cristallin de 1,1 GPM, de 1,6 GPS et de 0,01 à 1,99 % de 1,1 GPS et consiste à introduire une solution concentrée de 1,1 GPM, de 1,6 GPS et de 1,1 GPS avec des amorces cristallines de glucopyranosyl-alditols dans un extrudeur muni d'une zone de refroidissement fine et longue, puis à refroidir et à pétrir ladite solution aqueuse de glucopyranosyl-alditols en présence de germes cristallins, de manière à produire un magma de glucopyranosyl-alditols qui est extrudé continuellement depuis une buse.

WO 00/64916 décrit un procédé de cristallisation de 1,1-GPM, 1,6-GPS et 1,1-GPS consistant à placer une solution de glucopyranosyl-alditols en suspension dans un gaz et à sécher le matériel afin d'obtenir des particules cristallines de glucopyranosyl-alditols microcristallisés.

Il résulte de tout ce qui précède que les procédés de cristallisation de l'art antérieur présentent l'inconvénient d'être onéreux, complexes et de prendre beaucoup de temps.

L'invention a pour principal but de fournir un procédé de fabrication d'une poudre contenant des particules cristallines de glucopyranosyl-alditols qui est moins sensible aux inconvénients susmentionnés et qui permet d'obtenir efficacement une poudre contenant des particules cristallines de glucopyranosyl-alditols ayant les propriétés souhaitées.

Le procédé de fabrication de particules cristallines de glucopyranosyl-alditols conforme à l'invention ne nécessite pas une très forte concentration en glucopyranosyl-alditols, et ne nécessite pas non plus d'effort particulier pour contrôler ou mesurer précisément la température pendant l'étape de granulation / cristallisation.

De plus, le procédé de l'invention n'implique pas de formation de masse cuite, ni l'application d'une force de cisaillement ou de pétrissage, mais repose plutôt simplement sur un revêtement, une agglomération et une induction de la cristallisation concourants en permettant au mélange aggloméré de maturer à une température inférieure au point de fusion des glucopyranosyl-alditols, pour former des granulés solides.

Selon la présente invention, on fournit un procédé de fabrication d'une poudre contenant des particules cristallines de glucopyranosyl-alditols choisis dans le groupe constitué du 1-O-α-D-glucopyranosyl-D-mannitol, du 6-O-α-D-glucopyranosyl-D-sorbitol et du 1-O-α-D-glucopyranosyl-D-sorbitol, comprenant le mélange continu d'un sirop de glucopyranosyl-alditols ayant une teneur en matière sèche d'au moins 80 % en poids et une teneur en glucopyranosyl-alditols d'au moins 95 % en poids, de préférence d'au moins 98 % en poids sur la base de la matière sèche, le mélange étant effectué en dispersant simultanément le sirop de glucopyranosyl-alditols et des germes contenant des glucopyranosyl-alditols dans un récipient rotatif ouvert contenant des granulés à base de glucopyranosyl-alditols, ce par quoi le sirop de glucopyranosyl-alditols et les germes contenant des glucopyranosyl-alditols sont mélangés à la surface des granulés à base de glucopyranosyl-alditols contenus dans le récipient, la récupération des granulés à base de glucopyranosyl-alditols depuis le récipient et la cristallisation des glucopyranosyl-alditols contenus dans lesdits granulés, les granulés à base de glucopyranosyl-alditols dans le récipient étant maintenus en mouvement par la rotation du récipient.

Lors de la réalisation du procédé de l'invention, le sirop de glucopyranosyl-alditols est introduit de préférence dans le récipient sous une forme subdivisée par exemple sous forme de gouttes ou de globules, de jets ou de faisceaux de jets.

Selon une méthode préférée de réalisation du procédé susmentionné, un sirop de glucopyranosyl-alditols, ayant une teneur en matière sèche d'au moins 80 % en poids, est porté à une température d'au moins 90°C et est mélangé continuellement dans un récipient avec des germes contenant des glucopyranosyl-alditols, le rapport germes/ sirop, les dimensions, l'orientation de l'axe de rotation et la vitesse de rotation du récipient étant choisis de telle façon que le produit récupéré depuis le récipient apparaît sous la forme de granulés d'un diamètre moyen d'environ 100 à 50.000 µm.

Le procédé de l'invention peut être réalisé dans un appareil comprenant :
- un récipient rotatif ouvert, avec un axe de rotation pouvant être incliné horizontalement,
- un moyen d'apporter à une zone située à l'intérieur du récipient, au-dessus de la masse qui le remplit partiellement, et d'y disperser une partie de sirop de glucopyranosyl-alditols, de préférence subdivisé en les formes mentionnées ci-dessus et quelques germes contenant du glucopyranosyl-alditols, et
- un moyen d'assurer le mélange du sirop de glucopyranosyl-alditols et des germes contenant des glucopyranosyl-alditols à la surface de la masse en mouvement remplissant partiellement le récipient.

Les granulés à base de glucopyranosyl-alditols sont récupérés de préférence par débordement à la sortie du récipient et peuvent être maturés pour augmenter leur cristallinité par le transfert des granulés dans un cylindre rotatif de caractéristiques dimensionnelles telles que la durée du séjour des granulés provenant du récipient est suffisante pour assurer la cristallisation des glucopyranosyl-alditols.

Les granulés peuvent ensuite être transférés dans un séchoir pour diminuer l'humidité résiduelle et ensuite dans un moyen de broyage et de tamisage.

Lors de la réalisation de procédé de l'invention, un sirop de glucopyranosyl-alditols, du type disponible dans le commerce, et dont la matière sèche est d'au moins 70 %, et ayant une teneur en glucopyranosyl-alditols d'au moins 95 % en poids, de préférence d'au moins 98 % en poids sur la base de la matière sèche, est dispersé à une température d'environ 80°C à l'intérieur d'un récipient rotatif ouvert sous forme d'un réservoir ou d'un tambour ouvert ayant un fond essentiellement plat, dont l'axe de rotation peut être incliné sur un plan horizontal d'un angle de 25 à 45°.

Une buse d'atomisation pneumatique a été avantageusement utilisée pour pulvériser le sirop aqueux sur le lit rotatif de matériaux de germes contenant des glucopyranosyl-alditols, dans le granulateur à échelle pilote.

En ce qui concerne le transfert des germes contenant des glucopyranosyl-alditols dans le mélange susmentionné et dont les particules constituantes servent de germes contenant des glucopyranosyl-alditols cristallisés, on préfère une taille de particules d'environ 100 µm.

Le rapport pondéral dans le mélange constitué de germes contenant des glucopyranosyl-alditols et de sirop de glucopyranosyl-alditols est d'environ 4/1, mais peut être réduit à 2/1, voire à 1/1, en fonction des performances souhaitées.

Le mélange est effectué à la surface de la masse en mouvement remplissant partiellement le récipient. Le mouvement en question rappelle une masse de pilules à l'intérieur d'une machine de fabrication de pilules, et on a constaté que des granulés qui sont de plus en plus gros sont formés, les granulés les plus grands ayant tendance à venir à la surface de la masse en mouvement.

Les granulés de glucopyranosyl-alditols ainsi obtenus sont ensuite maturés pour augmenter leur cristallinité.

Cette étape de maturation peut être réalisée en conservant les granulés en mouvement à une température inférieure au point de fusion des granulés, préférablement à une température de 50 à 90°C, pendant de 20 minutes à 2 heures dans un courant d'air.

Le produit granulé est ensuite séché afin d'obtenir une humidité résiduelle comprise entre 2 et 10 %.

Les granulés peuvent ensuite être broyés jusqu'à la taille de particules requise et ensuite triés par tamisage ; les particules éliminées par tamisage peuvent ensuite être avantageusement recyclées vers le récipient susmentionné pour une utilisation comme germes contenant des glucopyranosyl-alditols.

La poudre résultante contenant des particules cristallines de glucopyranosyl-alditols selon l'invention est caractérisée par le fait qu'elle présente une valeur de compression, selon le test A, qui est supérieure à 220 N, de préférence comprise entre 250 et 400 N, et plus préférentiellement encore comprise entre 280 et 350 N.

La compressibilité de ladite poudre contenant des particules cristallines de glucopyranosyl-alditols est déterminée selon le test A suivant, décrit dans le brevet EP 220.103 dont la société Demanderesse est titulaire. Ce test A consiste à mesurer la force, exprimée en Newton, qui est représentative de la compressibilité d'une poudre contenant des particules cristallines de glucopyranosyl-alditols présentant, après broyage et tamisage, une taille de particules comprises entre 250 et 1250 µm. L'appareil de mesure utilisé est un Duromètre ERWEKA TBH 30 GMD.

Cette force traduit donc ici la résistance à l'écrasement d'un comprimé qui est cylindrique à faces convexes (rayon de courbure de 13 mm), d'un diamètre de 13 mm, d'une épaisseur de 6 mm et d'un poids de 0,845 g, soit d'une masse volumique apparente de 1,48 g/ml.

Les résultats montrent que la poudre contenant des particules cristallines de glucopyranosyl-alditols conforme à l'invention présente une valeur de compressibilité élevée, qui à la connaissance de la société Demanderesse, n'a encore jamais été décrite.

Les déterminations de la densité aérée, de la densité tassée et de l'angle de talus sont réalisées à l'aide d'un appareil commercialisé par la société HOSOKAWA sous la marque POWDER TESTER en appliquant la méthode recommandée par le constructeur.

Dans ces conditions, la poudre contenant des particules cristallines de glucopyranosyl-alditols, de taille de particules comprise entre 250 et 1250 *µ*m, présente une densité aérée comprise entre 0,700 et 0,750 g/ml, une densité tassée également comprise entre 0,700 et 0,750 g/ml et un angle de talus compris entre 42 et 45°.

L'exemple suivant illustre la préparation de la poudre contenant des particules cristallines de glucopyranosyl-alditols par l'utilisation du procédé selon l'invention.

### Exemple

On prépare une suspension de glucopyranosyl-alditols (composé A) à 70 % de matière sèche et on la place dans un récipient d'évaporation pour obtenir un sirop de glucopyranosyl-alditols ayant une teneur en matière sèche d'environ 85 % (la teneur en glucopyranosyl-alditols du composé A est présentée dans le tableau 1 ci-dessous, exprimée en poids sec sur la totalité des composants du mélange).

Ce sirop de glucopyranosyl-alditols est placé dans un réservoir de stockage à une température d'environ 135°C, à partir duquel il est continuellement prélevé au moyen d'une pompe qui assure sa dispersion sous forme de globules au moyen d'une buse.

Simultanément à la dispersion dudit sirop de glucopyranosyl-alditols, on introduit continuellement du matériau de germes supplémentaire dans le granulateur à échelle pilote pour réaliser un rapport pondéral de germes/sirop d'environ 4 parties de germes pour 1 partie de sirop de glucopyranosyl-alditols.

Les germes sont obtenus par le recyclage continu d'une fraction du matériau solidifié étant produit.

Des particules d'un solide de glucopyranosyl-alditols cristallin quelconque peuvent être utilisées pour fournir des germes pour la granulation initiale.

Aucun effort particulier n'est entrepris pour contrôler la température du granulateur (qui reste de l'ordre de 90°C).

Le granulateur tourne à une vitesse d'environ 6,5 tours par minute, son inclinaison étant de 30°, ce qui permet d'obtenir des granulés ayant un diamètre moyen d'environ 100 *µ*m à 50.000 µm.

Après cette étape de granulation, lesdits granulés sont maturés par l'achèvement de la cristallisation dans un dispositif de maturation (tambour rotatif allongé).

Les granulés maturés ainsi obtenus sont soumis à un broyage grossier puis séchés dans un lit fluidisé en utilisant de l'air à 60°C pendant 30 minutes.

Après le séchage, ils apparaissent sous la forme d'une poudre contenant environ 6 % d'humidité résiduelle (valeur déterminée selon la méthode KARL FISHER).

A la sortie du transporteur à rouleaux du lit fluidisé, la poudre séchée est conduite à une installation classique de broyage. La poudre séchée est ensuite tamisée et une partie en est recyclée comme germes contenant des glucopyranosyl-alditols vers le granulateur.

La poudre restante est la poudre contenant des particules cristallines de glucopyranosyl-alditols selon l'invention (composé B).

Le tableau 1 suivant présente les compositions en glucopyranosyl-alditols des composés A et B, ainsi que le résultat des mesures des paramètres DSC (Température de fusion et enthalpie).

**Tableau 1**

| | Composé A | Composé B |
|---|---|---|
| 1,1 GPS (% en poids sur sec) | 0,3 | 0,3 |
| 1,1 GPM (% en poids sur sec) | 48,3 | 48,1 |
| 1,6 GPS (% en poids sur sec) | 50,7 | 50,2 |
| Analyse DSC | | |
| ler pic d'endotherme | | |
| - Température de fusion (°C) | 95,39 | 99,25 |
| - Enthalpie (J/g) | 34,28 | 81,26 |
| 2ème pic d'endotherme | | |
| - Température de fusion (°C) | 146,24 | 125,23 |
| - Enthalpie (J/g) | 116,20 | 54, 10 |
| Humidité résiduelle | 2,5 | 6,0 |

La granulation du composé A par le procédé conforme à l'invention ne modifie en rien la composition chimique en glucopyranosyl-alditols.

Cependant, elle conduit à la modification du profil thermique de fusion du composé B, qui se traduit par la réduction de manière significative à la fois du point de fusion et de l'enthalpie du deuxième pic d'endotherme du composé B, et qui s'accompagne également d'une légère diminution de l'enthalpie du premier pic d'endotherme dudit composé B.

Le tableau 2 suivant présente les résultats des mesures de compressibilité, de densité aérée et densité tassée, d'angle de talus et d'écoulement des composés A et B.

**Tableau 2**

| | Composé A | Composé B |
|---|---|---|
| Compressibilité selon le test A (Newton) | 26 | 317 |
| Densité aérée (g/ml) | 0,936 | 0,729 |
| Densité tassée (g/ml) | 0,936 | 0,729 |
| Angle de talus (°) | 41 | 44 |

Le procédé selon l'invention confère à la poudre contenant des particules cristallines de glucopyranosyl-alditols ainsi obtenue une densité plus faible que le produit de départ, et surtout de remarquables propriétés de compression.

## Revendications

1. Procédé de fabrication d'une poudre contenant des particules cristallines de glucopyranosyl-alditols choisis dans le groupe constitué du 1-O-α-D-glucopyranosyl-D-mannitoi, du 6-O-α-D-glucopyranosyl-D-sorbitol et du 1-O-α-D-glucopyranosyl-D-sorbitol, comprenant le mélange continu d'un sirop de glucopyranosyl-alditols ayant une teneur en matière sèche d'au moins 80 % en poids et une teneur en glucopyranosylalditols d'au moins 95 % en poids, de préférence d'au moins 98 % en poids sur la base de la matière sèche, le mélange étant effectué en dispersant simultanément le sirop de glucopyranosyl-alditols et des germes contenant des glucopyranosyl-alditols dans un récipient rotatif ouvert contenant des granulés à base de glucopyranosyl-alditols, ce par quoi le sirop de glucopyranosyl-alditols et les germes contenant des glucopyranosyl-alditols sont mé.lanqés à la surface des granulés à base de glucopyranosyl-alditols contenus dans le récipient, la récupération des granulés à base de glucopyranosylalditols depuis le récipient et la cristallisation des glucopyranosyl-alditols contenus dans lesdits granulés, les granulés à base de glucopyranosyl-alditols dans le récipient étant maintenus en mouvement par la rotation du récipient.

2. Procédé selon la revendication 1, dans lequel le sirop de glucopyranosyl-alditols est introduit dans le récipient sous forme subdivisée.

3. Procédé selon la revendication 2, dans lequel le sirop de glucopyranosyl-alditols est introduit sous forme de gouttes.

4. Procédé selon la revendication 2, dans lequel le sirop de glucopyranosyl-alditols est introduit sous forme de jets.

5. Procédé selon l'une quelconque des revendications 1 à 4, dans lequel l'axe de rotation du récipient est incliné par rapport à l'horizontale.

6. Procédé selon l'une quelconque des revendications 1 à 5, dans lequel les granulés à base de glucopyranosyl-alditols sont récupérés par débordement à la sortie du récipient.

7. Procédé selon l'une quelconque des revendications 1 à 6, dans lequel des granulés d'un diamètre de 100 à 50 000 um sont récupérés.

8. Procédé selon la revendication 1, dans lequel un sirop de glucopyranosyl-alditols, ayant une teneur en matière sèche d'au moins 80 % en poids, est porté à une température d'au moins 30 °: et est mélangé continuellement avec des germes contenant des glucopyranosyl-alditols, le rapport germes/sirop, les dimensions, l'orientation de l'axe de rotation et la vitesse de rotation du récipient étant choisis de telle façon que le produit récupéré depuis le récipient apparait sous la forme de granulés ayant un diamètre de 100 à 50 000 µm.

9. Procédé selon la revendication 8, dans lequel le rapport germes/sirop n'est pas supérieur à environ 4/1.

10. Procédé selon l'une quelconque des revendications 1 à 9, dans lequel le récipient est sous la forme d'un réservoir ou d'un tambour ouvert ayant un fond essentiellement plat.

11. Procédé selon la revendication 10, dans lequel l'axe de rotation du récipient fait un angle de 25 à 45° par rapport à l'horizontale.

12. Procédé selon l'une quelconque des revendications 1 à 11, dans lequel les granulés de glucopyranosyl-alditols récupérés sont maturés afin d'augmenter leur cristallinité en maintenant les granulés à une température de 50 à 90°C pendant de 20 min à 2 heures tout en maintenant les granulés en mouvement dans un courant d'air.

13. Procédé selon la revendication 12, dans lequel les granulés de glucopyranosyl-alditols maturés récupérés sont broyés et séchés afin d'obtenir une humidité résiduelle comprise entre 2 et 10 %.

14. Poudre contenant des particules cristallines de glucopyranosyl-alditols obtenue par un procédé selon l'une quelconque des revendications 1 à 13.

15. Poudre contenant des particules cristallines de glucopyranosyl-alditols selon la revendication 14, **caractérisée en ce qu'**elle présente une valeur de compression, selon un test A, supérieure à 220 N, de peférence comprise entre 250 et 400 N, et plus préférentiellement encore comprise entre 280 et 350 N, ledit test A consistant à mesurer la force, exprimée en Newton, qui est représentative de la compressibilité d'une poudre de glucopyranosyl-alditols présentant, après broyage et tamisage, une taille de particules comprises entre 250 et 1250 µm, et qui est nécessaire pour provoquer l'écrasement d'un comprimé préparé à partir de ladite poudre, c'est-à-dire pour provoquer l'apparition de lignes de rupture au sein de la masse constitutive de celui-ci ; cette force traduisant donc la résistance à l'écrasement du comprimé qui est cylindrique à faces convexes d'un rayon de courbure de 13 mm, d'un diamètre de 13 mm, d'une épaisseur de 6 mm et d'un poids de 0,845 g, c'est-à-dire d'une masse volumique apparente de 1,48 g/ml ; ladite force étant exercée contre la surface périphérique du comprimé en direction de l'axe de révolution de celui-ci au moyen d'une butée mobile s'appliquant contre ladite surface le long d'une génératrice, ledit comprimé étant par ailleurs immobilisé contre une butée fixe appliquée également contre la surface périphérique du comprimé le long d'une génératrice diamétralement opposée à celle contre laquelle s'applique la butée mobile.

16. Poudre contenant des particules cristallines de glucopyranosyl-alditols selon la revendication 15, présentant, selon ia méthode HOSOKAWA, une densité aérée comprise entre 0,700 et 0,750 g/ml et une densité tassée comprise entre 0,700 et 0,750 g/ml.

17. Procédé de fabrication de comprimées par l'utilisation d'une poudre contenant des particules cristallines de glucopyranosyl-alditols telles que définies à l'une quelconque des revendications 14 à 16 ou pouvant être obtenue par un procédé tel que défini à l'une quelconque des revendications 1 à 13.

18. Procédé de fabrication de confiseries par l'utilisation d'une poudre contenant des particules cristallines de glucopyranosyl-alditols selon l'une quelconque des revendications 14 à 16 ou pouvant être obtenue par un procédé tel que défini à l'une quelconque des revendications 1 à 13.

## Patentansprüche

1. Verfahren zur Herstellung eines Pulvers, enthaltend kristalline Partikel von Glucopyranosyl-Alditolen ausgewählt aus der Gruppe bestehend aus 1-0- α -D-glucopyranosyl-D-mannitol, 6-O- α -D-glucopyranosyl-D-sorbitol und 1-0- α -D-glucopyranosyl-D-sorbitol, umfassend das kontinuierliche Mischen eines Glucopyranosyl-Alditolsirups mit einem Trockenmassegehalt von mindestens 80 Gewichts-% und einem Gehalt an Glucopyranosyl-Alditolen von mindestens 95 Gewichts-%, bevorzugt von mindestens 98 Gewichts-%, bezogen auf das Trockenmaterial, wobei das Mischen durchgeführt wird, indem der Glucopyranosyl-Alditolsirup und Keime, die Glucopyranosyl-Alditole enthalten gleichzeitig in einem offenen Rotationsbehälter, welcher Körnchen auf Basis von Glucopyranosyl-Alditolen enthält, dispergiert werden, wodurch der Glucopyranosyl-Alditolsirup und die Keime, die Glucopyranosyl-Alditole enthalten, auf der Oberfläche der Körnchen auf Basis von Glucopyranosyl-Alditolen, die in dem Behälter enthalten sind, gemischt werden, das Zurückgewinnen der Kömchen auf Basis von Glucopyranosyl-Alditolen aus dem Behälter und das Kristallisieren der in den Körnchen enthaltenen Glucopyranosyl-Alditole, wobei die Körnchen auf Basis von Glucopyranosyl-Alditolen in dem Behälter durch Rotation des Behälters in Bewegung gehalten werden.

2. Verfahren gemäß Anspruch 1, wobei der Glucopyranosyl-Alditolsirup in den Behälter in unterteilter Form eingeführt wird.

3. Verfahren gemäß Anspruch 2, wobei der Glucopyranosyl-Alditolsirup in Form von Tropfen eingeführt wird.

4. Verfahren gemäß Anspruch 2, wobei der Glucopyranosyl-Alditolsirup in Form von Strahlen eingeführt wird.

5. Verfahren gemäß einem der Ansprüche 1 bis 4, wobei die Rotationsachse des Behälters in Bezug auf die Horizontale geneigt ist.

6. Verfahren gemäß einem der Ansprüche 1 bis 5, wobei die Körnchen auf Basis von Glucopyranosyl-Alditolen durch Überlaufen am Auslass des Behälters zurückgewonnen werden.

7. Verfahren gemäß einem der Ansprüche 1 bis 6, wobei Körnchen mit einem Durchmesser von 100 bis 50.000 µm zurückgewonnen werden.

8. Verfahren gemäß Anspruch 1, wobei ein Glucopyranosyl-Alditolsirup mit einem Trockenmassegehalt von mindestens 80 Gewichts-% auf einer Temperatur von mindestens 80°C geheizt wird und kontinuierlich mit Keimen, die Glucopyranosyl-Alditole enthalten, gemischt wird, wobei das Verhältnis Keime/Sirup, die Dimensionen, die Orientierung der Rotationsachse und die Rotationsgeschwindigkeit des Behälters so gewählt sind, dass das aus dem Behälter zurückgewonnene Produkt in Form von Körnchen mit einem Durchmesser von 100 bis 50.000 µm auftritt.

9. Verfahren gemäß Anspruch 8, wobei das Verhältnis Keime/Sirup nicht größer als etwa 4/1 ist.

10. Verfahren gemäß einem der Ansprüche 1 bis 9, wobei der Behälter ein Reservoirs oder eine offene Trommel mit einem im Wesentlichen flachen Boden ist.

11. Verfahren gemäß Anspruch 10, wobei die Rotationsachse des Behälters einen Winkel von 25 bis 45° in Bezug auf die Horizontale bildet.

12. Verfahren gemäß einem der Ansprüche 1 bis 11, wobei man die zurückgewonnenen Glucopyranosyl-Alditol-Körnchen reifen lässt, um ihre Kristallinität zu erhöhen, indem man die Körnchen bei einer Temperatur von 50 bis 90°C für 20 min bis 2 Stunden hält, wobei die Körnchen in einem Luftstrom in Bewegung gehalten werden.

13. Verfahren gemäß Anspruch 12, wobei die gereiften, zurückgewonnenen Körnchen von Glucopyranosyl-Alditolen zerkleinert und getrocknet werden bis zu einer Restfeuchtigkeit von zwischen 2 und 10%.

14. Pulver, enthaltend kristalline Partikel von Glucopyranosyl-Alditolen, erhalten durch ein Verfahren gemäß einem der Ansprüche 1 bis 13.

15. Pulver, enthaltend kristalline Partikel von Glucopyranosyl-Alditolen gemäß Anspruch 14, mit einem Kompressionswert gemäß einem Test A von größer als 220 N, bevorzugt zwischen 250 und 400 N und noch stärker bevorzugt zwischen 280 und 350 N, wobei der Test A darin besteht, die Kraft, ausgedrückt in Newton, zu messen, die für die Kompressibilität eines Pulvers von Glucopyranosyl-Alditolen, nach Mahlen und Sieben, mit eine Teilchengröße von zwischen 250 und 1250 µm repräsentativ ist und die nötig ist, um das Brechen einer aus dem Pulver hergestellten Tablette herbeizuführen, das heißt um das Auftreten von Bruchlinien in der daraus bestehenden Masse hervorzurufen, wobei die Kraft also die Bruchfestigkeit der Tablette, die zylindrisch ist, mit konvexen Flächen mit einem Krümmungsradius von 13 mm, mit einem Durchmesser von 13 mm, einer Dicke von 6 mm und einem Gewicht von 0,845 g, das heißt einer Rohdichte von 1,48 g/ml, zum Ausdruck bringt, wobei die Kraft gegen die Umfangsfläche der Tablette in Richtung deren Rotationsachse mittels eines beweglichen Anschlags, der gegen die Fläche entlang einer Erzeugenden angewendet wird, ausgeübt wird, wobei die Tablette außerdem gegen einen stationären Anschlag immobilisiert ist, der gleichermaßen gegen die Umfangsfläche der Tablette entlang einer Erzeugenden angewendet ist, die sich diametral gegenüber derjenigen befindet, gegen die der bewegliche Anschlag angewendet wird.

16. Pulver, enthaltend kristalline Partikel von Glucopyranosyl-Alditolen gemäß Anspruch 15, welches gemäß dem HOSOKAWA-Verfahren eine Schüttdichte zwischen 0,700 und 0,750 g/ml und eine gepackte Dichte zwischen 0,700 und 0,750 g/ml aufweist.

17. Verfahren zur Herstellung von Tabletten unter Verwendung eines Pulvers, enthaltend kristalline Partikel von Glucopyranosyl-Alditolen gemäß einem der Ansprüche 14 bis 16 oder erhältlich durch ein Verfahren gemäß einem der Ansprüche 1 bis 13.

18. Verfahren zur Herstellung von Süßwaren unter Verwendung eines Pulvers, enthaltend kristalline Partikel von Glucopyranosyl-Alditolen gemäß einem der Ansprüche 14 bis 16 oder erhältlich durch ein Verfahren gemäß einem der Ansprüche 1 bis 13.

## Claims

1. A method for making a powder containing crystalline particles of glucopyranosyl-alditols chosen in the group constituted of 1-O- α -D-glucopyranosyl-D-mannitol, 6-O- α -D-glucopyranosyl-D-sorbitol and 1-O- α -D-glucopyranosyl-D-sorbitol, comprising continuous mixing a glucopyranosyl-alditol syrup having a dry matter content of at least 80% by weight and a glucopyranosyl-alditol content of at least 95% by weight, preferably of at least 98% by weight, based on dry matter, the mixing being carried out by simultaneously dispersing the glucopyranosyl-alditol syrup and germs containing glucopyranosyl-alditols in an open rotary container containing (glucopyranosyl-alditol)-based granules, whereby the glucopyranosyl--alditol syrup and the germs containing glucopyranosyl-alditols are mixed at the surface of the (glucopyranosyl-alditol)-based granules contained in the container, recovering the (glucopyranosyl-alditol)-based granules from the container and crystallizing the glucopyranosyl-alditols contained in said granules, the (glucopyranosyl-alditol)-based granules in the container being kept in motion by rotating the container.

2. The method according to claim 1, wherein the glucopyranosyl-alditol syrup is introduced into the container in a subdivided form.

3. The method according to claim 2, wherein the glucopyranosyl-alditol syrup is introduced as drops.

4. The method according to claim 2, wherein the glucopyranosyl-alditol syrup is introduced as jets.

5. The method according to one of claims 1 to 4, wherein the axis of rotation of the container is tilted relatively to the horizontal.

6. The method according to one of claims 1 to 5, wherein the (glucopyranosyl-alditol)-based granules are recovered by overflow at the outlet of the container.

7. The method according to one of claims 1 to 6, wherein granules with a diameter of 100 to 50,000 µm are recovered.

8. The method according to claim 1 wherein a glucopyranosyl-alditol syrup, with a dry matter content of at least 80% by weight, is brought to a temperature of at least 80°C and is continually mixed with germs containing glucopyranosyl-alditols, wherein the germs/syrup ratio, the dimensions, the orientation of the axis of rotation and the velocity of rotation of the container are selected so that the product recovered from the container appears as granules having a diameter of 100 to 50,000 µm.

9. The method according to claim 8, wherein the germs/syrup ratio is not greater than about 4/1.

10. The method according to claim 1, wherein the container is in the form of a tank or an open drum having an essentially flat bottom.

11. The method according to claim 10, wherein the axis of rotation of the container forms an angle of 25 to 45° relatively to the horizontal.

12. The method according to one of claims 1 to 11, wherein the recovered glucopyranosyl-alditol granules are matured so as to increase their crystallinity by maintaining the granules at a temperature of 50 to 90°C for 20 minutes to 2 hours while keeping the granules in motion in an air stream.

13. The method according to claim 12, wherein the matured recovered granules of glucopyranosyl-alditols are milled and dried in order to obtain a residual moisture of between 2 and 10%.

14. A powder containing crystalline particles of glucopyranosyl-alditols obtainable by a method according to one of claims 1 to 13.

15. A powder containing crystalline particles of glucopyranosyl-alditols according to claim 14 having a compression value, according to a test A, greater than 220 N, preferably of between 250 and 400 N, and still more preferentially between 280 and 350 N, wherein test A consists in measuring the strength, expressed in Newtons, typical of the compressibility of the of a powder of glucopyranosyl-alditols having, after milling and sieving, a particle size of between 250 and 1250 µm., and which is necessary to cause a tablet prepared from said powder to be crushed, i.e. to bring about the formation of rupture lines within the constituent mass thereof, this strength thus reflecting the resistance to crushing of said tablet, which is cylindrical with convex faces with 13 mm radius of curvature, with a diameter of 13 mm, a thickness of 6 mm and a weight of 0.845 g, i.e. having a specific gravity of 1.48 g/ml, the said strength being exerted against the peripheral surface of the tablet in the direction of the axis of revolution thereof by means of a mechanical movable stop applied against said surface along a generatrix, the said tablet being furthermore immobilized against a mechanical stationary stop also applied against the peripheral surface of the tablet along a generatrix, the latter being diametrically opposed to the generatrix against which the mechanical movable stop is applied.

16. A powder containing crystalline particles of glucopyranosyl-alditols according to claim 15, having, according to the HOSOKAWA method, an aerated density of between 0.700 and 0.750g/ml and a packed density of between 0.700 and 0.750 g/ml.

17. A method for making tablets by using a powder containing crystalline particles of glucopyranosyl-alditols defined in one of claims 14 to 16 or obtainable by a method according to one of claims 1 to 13.

18. A method for making confectionery by using a powder containing crystalline particles of gluccpyranosyl-alditols defined in one of claims 14 to 16 or obtainable by a method according to one of claims 1 to 13.
